# EUROPEAN PATENT APPLICATION

(11) **EP 1 710 301 A1**
(43) Date of publication of application: **11.10.2006**
(21) Application number: 05007563.9
(22) Date of filing: 06.04.2005
(51) Int. Cl.: C12N 9/04, C12N 1/20, G01N 33/50, A61K 39/04, A61P 31/06

(54) **L-gulonolactone dehydrogenase of Mycobacterium tuberculosis**

(71) Applicant: Wolucka, Beata A., 1200 Brussels (BE); Pasteur Institute of Brussels, 1180 Brussels (BE)
(72) Inventor: Wolucka, Beata A., 1180 Brussels (BE)

(57) **Abstract**

The present invention relates to a previously unknown enzyme of the *Mycobacterium tuberculosis* complex, an L-gulonolactone dehydrogenase, and in particular, to an enzyme which catalyzes the oxidation of L-gulonolactone to L-ascorbic acid (L-threo-2-hexenono-1,4-lactone; vitamin C).

SEQUENCE LISTING

<110> PASTEUR INSTITUTE OF BRUSSELS
WOLUCKA, Beata A.

<120> L-GULONOLACTONE DEHYDROGENASE OF MYCOBACTERIUM TUBERCULOSIS

<130> BW_VitC_Gulox

<160> 2

<170> PatentIn version 3.1

<210> 1
<211> 1287
<212> DNA
<213> Mycobacterium tuberculosis

<300>
<308> GenBank:BX842577
<309> 2004-12-02
<313> (273904)..(275190)

<400> 1
atgagcccga tatggagtaa ttggcctggt gagcaagtct gcgcgccgtc ggcgatcgta 60
cggccgacct cggaggctga gctggccgac gtgatcgcgc aggcggcgaa aagaggcgag 120
cgggtacgcg cggttggcag cgggcattcg tttaccgaca tcgcctgcac ggacggggtc 180
atgatcgaca tgaccggcct gcagcgggtc ctcgacgtgg accagccgac tggcctggtg 240
acggtcgagg ggggcgcaaa gctacgtgcg ctgggacccc aattggcgca acgacggctc 300
ggcctggaga accagggtga cgtggatccc caatccatca ccggcgcgac cgcgaccgcg 360
acgcacggaa ccggggtgcg tttccagaat ctgtcggcgc ggatcgtttc gctgcggctg 420
gtcaccgcgg gcggggaagt gctcagtctg tccgaaggtg acgattacct ggcggcacgg 480
gtttccctcg gcgcgctagg agtgatctca caggtcaccc tgcagacggt tccgctattc 540
acgttgcatc gccatgatca gcgacgctcg ctggcgcaga cgctggagcg cctcgacgag 600
ttcgtggacg gtaatgacca tttcgagttt ttcgtattcc cttacgcaga taaggcgttg 660
acgcgcacca tgcatcgcag tgacgagcag cccaaaccca cgcccgggtg gcagcgcatg 720
gtcggcgaga acttcgagaa cgggggattg agcctgatct gccagaccgg ccgtcgtttt 780
cctagtgtgg cgccgcgact gaaccgcctg atgacgaaca tgatgtcgtc ctccaccgtg 840
caagaccgcg cctacaaggt ctttgcgacc caacgcaagg tcaggttcac cgagatggag 900

tacgcgatcc cgcgtgaaaa cgggcgcgag gcgctccagc gtgtcatcga ccttgtgcgc 960
cgtcgcagct tgccgatcat gtttccgatt gaggtgcgat tctccgcccc cgacgattcc 1020
ttcctgtcga ccgcatatgg gcgcgacact tgctacatcg cggttcatca atacgccggt 1080
atggagttcg aaagctactt ccgcgccgtc gaggagatca tggacgacta cgccggtcgg 1140
ccacactggg gtaaacgtca ctatcagacc gccgccacgc ttcgtgagcg ctatccgcag 1200
tgggatcggt tcgccgcggt tcgcgatcgc ctcgatccgg accgggtgtt tctcaacgac 1260
tacacccggc gcgttctcgg tccctga 1287

<210> 2
<211> 428
<212> PRT
<213> Mycobacterium tuberculosis

<300>
<308> UniProt/TrEMBL:O06804
<309> 1997-07-01
<313> (1)..(428)

<400> 2
Met Ser Pro Ile Trp Ser Asn Trp Pro Gly Glu Gln Val Cys Ala Pro
1               5                   10                  15

Ser Ala Ile Val Arg Pro Thr Ser Glu Ala Glu Leu Ala Asp Val Ile
            20                  25                  30

Ala Gln Ala Ala Lys Arg Gly Glu Arg Val Arg Ala Val Gly Ser Gly
        35                  40                  45

His Ser Phe Thr Asp Ile Ala Cys Thr Asp Gly Val Met Ile Asp Met
    50                  55                  60

Thr Gly Leu Gln Arg Val Leu Asp Val Asp Gln Pro Thr Gly Leu Val
65                  70                  75                  80

Thr Val Glu Gly Gly Ala Lys Leu Arg Ala Leu Gly Pro Gln Leu Ala
                85                  90                  95

Gln Arg Arg Leu Gly Leu Glu Asn Gln Gly Asp Val Asp Pro Gln Ser
            100                 105                 110

Ile Thr Gly Ala Thr Ala Thr Ala Thr His Gly Thr Gly Val Arg Phe
        115                 120                 125

Gln Asn Leu Ser Ala Arg Ile Val Ser Leu Arg Leu Val Thr Ala Gly
    130                 135                 140

Gly Glu Val Leu Ser Leu Ser Glu Gly Asp Asp Tyr Leu Ala Ala Arg
145                 150                 155                 160

Val Ser Leu Gly Ala Leu Gly Val Ile Ser Gln Val Thr Leu Gln Thr
                165                 170                 175

Val Pro Leu Phe Thr Leu His Arg His Asp Gln Arg Arg Ser Leu Ala
            180                 185                 190

Gln Thr Leu Glu Arg Leu Asp Glu Phe Val Asp Gly Asn Asp His Phe
        195                 200                 205

Glu Phe Phe Val Phe Pro Tyr Ala Asp Lys Ala Leu Thr Arg Thr Met
    210                 215                 220

His Arg Ser Asp Glu Gln Pro Lys Pro Thr Pro Gly Trp Gln Arg Met
225                 230                 235                 240

Val Gly Glu Asn Phe Glu Asn Gly Gly Leu Ser Leu Ile Cys Gln Thr
                245                 250                 255

Gly Arg Arg Phe Pro Ser Val Ala Pro Arg Leu Asn Arg Leu Met Thr
            260                 265                 270

Asn Met Met Ser Ser Ser Thr Val Gln Asp Arg Ala Tyr Lys Val Phe
        275                 280                 285

Ala Thr Gln Arg Lys Val Arg Phe Thr Glu Met Glu Tyr Ala Ile Pro
    290                 295                 300

Arg Glu Asn Gly Arg Glu Ala Leu Gln Arg Val Ile Asp Leu Val Arg
305                 310                 315                 320

Arg Arg Ser Leu Pro Ile Met Phe Pro Ile Glu Val Arg Phe Ser Ala
                325                 330                 335

Pro Asp Asp Ser Phe Leu Ser Thr Ala Tyr Gly Arg Asp Thr Cys Tyr
            340                 345                 350

Ile Ala Val His Gln Tyr Ala Gly Met Glu Phe Glu Ser Tyr Phe Arg
        355                 360                 365

Ala Val Glu Glu Ile Met Asp Asp Tyr Ala Gly Arg Pro His Trp Gly
    370                 375                 380

Lys Arg His Tyr Gln Thr Ala Ala Thr Leu Arg Glu Arg Tyr Pro Gln
385                 390                 395                 400

Trp Asp Arg Phe Ala Ala Val Arg Asp Arg Leu Asp Pro Asp Arg Val
                405                 410                 415

Phe Leu Asn Asp Tyr Thr Arg Arg Val Leu Gly Pro
            420                 425

## Description

The present invention relates to a previously unknown enzyme of *Mycobacterium tuberculosis* and of *Mycobacterium bovis,* Lgulono-1,4-lactone dehydrogenase, and, in particular, to an enzyme which catalyzes the oxidation of L-gulono-1,4-lactone to L-ascorbic acid (vitamin C; L-threo-2-hexenono-1,4-lactone) in these bacteria. The enzyme of the invention can be used for the microbiological manufacture of L-ascorbic acid, for the production of transgenic plants with increased vitamin C content, for the production of attenuated live vaccines in the prevention of tuberculosis, and as a target for new antituberculosis drugs.

Vitamin C (L-ascorbic acid) is an important metabolite of plants and animals. It functions as an antioxidant, an enzyme cofactor and a cell-signaling modulator, in a wide array of crucial physiological processes, including biosynthesis of hydroxyproline-containing proteins/peptides, hormones and secondary metabolites; stress resistance; photoprotection; cell division and growth. Humans and some animals (including other primates and guinea pigs) are unable to synthesize Lrascorbic acid and are completely dependent upon ingested food, mainly of plant origin, for the acquisition of the vitamin. A lack of vitamin C in the diet leads to scurvy. Fungi apparently do not contain L-ascorbic acid but its 5-carbon homolog, D-erythroascorbic acid (Loewus, 1999).

Several attempts have been made to create genetically modified plants with an increased L-ascorbic acid content, with only limited success (Jain and Nessler, 2000; Agius et al., 2003; Chen et al., 2003). Further research is needed in order to better understand the mechanisms of ascorbate homeostasis in plants.

Plants synthesize ascorbic acid *via de novo* and salvage pathways (Valpuesta and Botella, 2004), whereas a *de novo* pathway involving UDP-D-glucuronic acid is operating in animals (Linster and Van Schaftingen, 2003). L-Gulono-1,4-lactone is a direct precursor of vitamin C in animals (Nishikimi and Yagi, 1996), but also in plants (Wolucka and van Montagu, 2003) and in algae (Shigeoka et al., 1979). The oxidation of L-gulono-1,4-lactone to L-ascorbic acid in animals is catalyzed by an oxygen-dependent enzyme, L-gulono-1,4-lactone oxidase (Nishikimi, 1979; Kiuchi et al., 1982). In plants (Wolucka and van Montagu, 2003) and algae (Shigeoka et al., 1979), the oxidation involves an L-gulono-1,4-lactone dehydrogenase that can use cytochrome c, as a direct electron acceptor but not molecular oxygen.

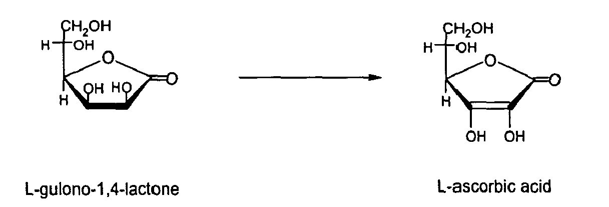

The animal and plant L-gulonolactone oxidases and dehydrogenases, respectively, are also active towards the L-galactono-1,4-lactone substrate.

Two apparently different L-galactono-1,4-lactone oxidase activities were detected in yeasts. One of the enzymes catalyzes the oxidation of both L-galactono-1,4-lactone and

L-gulono-1,4-lactone (Nishikimi et al., 1978) and also of D-arabinono-1,4-lactone which is its natural substrate in the pathway to D-erythroascorbic acid (Huh et al., 1994). The other enzyme was reported to be active with L-galactono-1,4-lactone but not with L-gulono-1,4-lactone (Bleeg et al., 1982).

The gene sequence encoding the rat L-gulono-1,4-lactone oxidase is known (Koshizaka et al., 1988). Humans have a nonfunctional gene for L-gulono-1,4-lactone oxidase (Nishikimi et al., 1994) and, therefore, are unable to synthesize L-ascorbic acid (vitamin C). In yeast, the gene encoding the D-arabinono-1,4-lactone oxidase (*ALO1*) (Huh et al., 1998) shows a significant homology with the rat hgulono-1,4-lactone oxidase and the highly specific plant L-galactono-1,4-lactone dehydrogenase (Ostergaard et al., 1997; Imai et al., 1998). Genes for L-gulono-1,4-lactone dehydrogenase isoenzymes of plants have not been identified yet (Wolucka and Van Montagu, 2003).

Despite an early report on L-ascorbic acid in *Mycobacterium tuberculosis* (Allaudeen and

Rhamakrishnan, 1971), there are no other reports on the presence of vitamin C in bacteria. The synthesis of L-ascorbic acid from exogenous L-gulono-1,4-lactone by growing and resting cells of *Gluconobacter oxydans* and of *Acetobacter suboxydans* was reported (Hoshino et al., 1993; Sugisawa et al., 1995). The enzyme responsible for the oxidation of L-gulono-1,4-lactone was an unspecific dehydrogenase that used also D-xylose, as substrate. The enzyme had low affinity for Lgulono-1,4-lactone. The protein was heteromeric and consisted of three different subunits. The corresponding polypeptides and genes have never been identified.

In the present invention, we identified the sequence SEQ ID NO : 1, cloned and expressed an L-gulono-1,4-lactone dehydrogenase of the *Mycobacterium tuberculosis* complex. Said sequence was already disclosed in the GenBank database (Accession #

BX842577) and classified as a probable oxidoreductase of unknown function. Surprisingly, we were able to demonstrate that the gene product comprising SEQ ID NO : 2, is a dehydrogenase that oxidizes L-gulono-1,4-lactone to L-ascorbic acid (vitamin C) and uses cytochrome c as an electron acceptor. The enzyme is unique because it has an absolute specificity to L-gulonolactone and no oxidase activity, and is encoded by only one gene. Accordingly, we have discovered that *Mycobacterium tuberculosis* and

*Mycobacterium bovis* can synthesize L-ascorbic acid (vitamin C).

Another aspect of the invention is a host cell transformed with a recombinant nucleic acid molecule encoding an amino acid sequence that is at least 70% identical to SEQ ID NO : 2. Preferably, said host cell is a bacterial cell or a plant cell.

Still another aspect of the invention is the use of L-gulono-1,4-lactone dehydrogenase to modulate vitamin C synthesis in said host cell.

*Mycobacterium tuberculosis* and the closely related *Mycobacterium bovis* belong to the *Mycobacterium tuberculosis* complex and both are etiological agents of human tuberculosis. There are about 8 million new cases of tuberculosis and more than 2 million deaths reported each year (http://www.who.int/gtb). The emergence of multi-drug resistant strains of *Mycobacterium tuberculosis,* the HIV pandemic and the variable efficacy of the current BCG vaccine have all contributed to a growing global tuberculosis problem. Therefore, there is an urgent need for new antituberculosis drugs as well as for improved live tuberculosis vaccines. BCG, an attenuated mutant of *M. bovis* that was isolated following serial subcultivations, is currently the only antituberculous vaccine available for use in humans, yet its precise mechanisms of attenuation have never been determined. Due to uncertain efficacy of BCG, particularly in tuberculosis-endemic countries, the development of improved tuberculosis vaccines has become an international research priority (Castanon-Arreola and Lopez-Vidal, 2004). A desirable trait in an effective live attenuated vaccine strain is its ability to persist within the host in a limited fashion in order to produce important protective antigens in vivo (McKenney et al., 1999). Rationally attenuated *M. tuberculosis* vaccine candidates have been obtained by deleting genes required for growth (nutrient-requiring or auxotrophic mutants) (Jackson et al., 1999; Hondalus et al., 2000; Sambandamurthy et al., 2002; Pavelka et al., 2003). However, the majority of these candidate vaccines failed to elicit an adequate protective immunity due to their inability to persist sufficiently long within the host tissues. In contrast, a highly attenuated pantothenate (vitamin B5) auxotroph of *M. tuberculosis,* which lacks two genes required to synthesize pantothenate, was reported to protect mice against tuberculosis (Sambandamurthy et al., 2002).

It was reported that the D-erythroascorbic acid-deficient *alo1*/*alo1* mutant of *Candida albicans* is attenuated and shows impaired growth (Huh et al., 2001). Similarly, deletions leading to the loss of L-ascorbic acid (vitamin C) would be expected to lead to attenuated virulence of an otherwise virulent mycobacterium in the *M. tuberculosis* complex.

Any of those strains would also be expected to sustain an infection in a mammal.

A preferred vitamin C deletion is of structural genes for enzymes involved in the biosynthesis of the vitamin, even more preferably the deletion of the sequence from *M*. *tuberculosis* provided herein as SEQ ID NO : 1.

The deletion can be made by genetic engineering, since such genetic methods allow precise control of the deletion being made. Various methods of making deletions in mycobacteria are known in the art. Non-limiting examples include specialized transduction (Bardarov et al., 2002), and sequential two-step recombinantion (Pelicic et al., 1997). In accordance with the present invention there may be employed conventional molecular biology, microbiology, and recombinant DNA techniques within the skill of the art. Such techniques are explained fully in the literature (Maniatis, Fritsch & Sambrook "Molecular Cloning: A Laboratory Manual", 1989; "Current Protocols in Molecular Biology" Volumes I-IV (Ausubel, R.M., ed.), 1997).

Strains with this deletion can be determined by any means in the art, preferably by molecular genetic means, for example by hybridization methods (e.g., Southern blot using a probe from the SEQ ID NO : 1 region) or by amplification methods (e.g., PCR using primers to amplify a portion of said region). The skilled atisan could identify analogous regions from other *M. tuberculosis* complex mycobacteria without undue experimentation. Those regions would be expected to have strong homology to SEQ ID NO : 1, at least 80% homologous to SEQ ID NO : 1.

In embodiment where the deletion is in a region controlling production of vitamin C or its precursors, the deletion can be in any genetic element leading to loss of production of the vitamin, including structural genes for enzymes involved in the biosynthesis of the vitamin, and genetic control elements such as promoters, enhancers, etc. Methods for determining whether a mycobacterium has deletions leading to the loss of production of vitamin C are within the scope of the art.

The scope of the present invention includes novel mycobacteria in the *M. tuberculosis* complex that are genetically engineered to comprise a deletion of a region controlling production of vitamin C. These mycobacteria include any in the *M. tuberculosis* complex, including *M. africanum, M. bovis, M. canetti, M. microti* and *M. tuberculosis.*

Preferred species are *M. bovis* and *M. tuberculosis,* since those species are the most important as causes of mammalian diseases, such as tuberculosis in humans and *M. bovis* infection in cows.

When constructing a live vaccine that is an attenuated pathogen due to a deletion, it is often desirable to include a second deletion, to better assure the safety of the vaccine (Sampson et al., 2004; Sambandamurthy et al., 2005). The second deletion preferably can also attenuate virulence of an otherwise virulent mycobacterium in the *M. tuberculosis* complex. The second deletion can be a deletion that would cause a prototrophic mycobacterium to be auxotrophic, or any other deletion that could improve the safety or efficacy of the mycobacterium in protecting against infection. Thus, in one embodiment, the invention is directed to mycobacteria in the *M*. *tuberculosis* complex which are genetically engineered to comprise more than one deletion. Preferably, each of the deletions is capable of individually attenuating virulence when engineered into a virulent mycobacterium in the *M. tuberculosis* complex.

The attenuated mycobacteria could be useful in methods and compositions for vaccinations of humans. Accordingly, in some embodiments, the present invention is directed to mycobacteria in the *M. tuberculosis* complex that are genetically engineered to comprise a deletion of a region controlling production of vitamin C. The *M*. *tuberculosis* preferably exhibits attenuated virulence in a mammal when conpared to the *M. tuberculosis* without the deletion.

In further embodiments, the invention is directed to tuberculosis vaccines comprising any of the above-described mycobacteria in the *M. tuberculosis* complex, in a pharmaceutically acceptable mixture. These vaccines are capable of protecting the mammal from challenge by a virulent *M. tuberculosis* complex mycobacterium. By vaccine is meant an agent used to stimulate the immune system of an individual so that protection is provided against an antigen not recognized as a self-antigen by the immune system.

Another aspect of the invention is the use of the mycobacterial L-gulonolactone dehydrogenase as a target for a rational designing of new antituberculosis drugs. Humans are deficient in L-gulonolactone oxidase because of several mutations in the corresponding gene (Nishikimi et al., 1994). Consequently, inhibitors of the mycobacterial L-gulono-1,4-lactone dehydrogenase would not be harmful for the human host. Inhibitors of L-gulonolactone oxidase are known in the art (Arrigoni et al., 1996).

### EXAMPLES

### Materials and methods to the examples

**Reagents.** GST-affinity resin was from Stratagene (Madison, WI). L-Gulono-1,4-lactone and cytochrome c was purchased from Sigma-Aldrich (St. Louis, MO). All reagents were of analytical grade.

**Plasmid construction.** The ORF corresponding to the mycobacterial L-gulonolactone dehydrogenase (1287 bp) was PCR amplified from the genomic DNA of *Mycobacterium tuberculosis* H37Rv. Oligonucleotide primers were designed with *att*B1 or *att*B2 site for the insertion into the GATEWAY donor vector pDONR201 (Invitrogen, Gaithersburg, MD) by homologous recombination. A sequence (GATGACGACGACAAG) corresponding to the enterokinase cleavage site (DDDDK) was included within the forward primer immediately upstream the start codon (ATG). Primers with the following sequences were synthesized by Proligo (Paris, France): 5forGulox (forward), 5'-GGGGACAAGTTTGTACAAAAAAGCAGGCTTCGATGACGACGACAAGATGAG CCCGATATGGAGTAATTGGCCT-3'; and 3revGulox (reverse), 5'-GGGGACCACTTTGTACAAGAAAGCTGGGTCTCAGGGACCGAGAACGCGCCG

GGTGTA-3'. The PCR product was cloned into the pDONR201 vector, and the resulting plasmid, pENTR_Gulox, was used to transfer the gene sequence into pDEST15 (Invitrogen) [glutathione S-transferase (GST)-tag N-termi nal fusion] by means of homologous recombination. The so obtained pDEST15_Gulox (GST-fusion) plasmid was used for expression of the protein in *Escherichia coli* BL21(DE3) cells.

**Heterologous expression of the recombinant L-gulono-1,4-lactone dehydrogenase.** *E. coli* cells carrying the pDEST15_Gulox plasmid were grown at 37 °C to an OD at 600 nm of 0.8 in 200 ml culture volume, and then isopropyl-β-D-thiogalactopyranoside was added to a final concentration of 0.4 mM for induction, and the fusion protein was produced for 3 hours. The cells were washed, resuspended in 3 volumes of 100 mM phosphate buffer (pH 7.3) containing 0.1% Triton X-100, 1 mM DTT, 1 mM PMSF and 20% glycerol, and sonicated. After centrifugation at 12000 rpm for 15 min, the supernatant was loaded on a GST-affinity column.

**GST-affinity chromatography of the recombinant L-gulono-1,4-lactone dehydrogenase of *Mycobacterium tuberculosis.*** A crude extract containing the GST-tagged dehydrogenase was applied to a 1-ml GST-affinity column equilibrated with 50 mM phosphate buffer (pH 7.3) containing 0.1% Triton X-100, 1 mM PMSF and 20% glycerol (buffer A). The column was washed with 15 volumes of buffer A and the recombinant dehydrogenase was eluted with 3 volumes of 10 mM glutathione (reduced form) in buffer A. Fractions containing the recombinant dehydrogenase were pooled, and glutathione was removed by gel filtration on a prepacked NAP-25 column (Amersham Pharmacia Biotech).

**Enterokinase cleavage of the GST-tagged L-gulono-1,4-lactone dehydrogenase. In** order to remove the GST-tag, an aliquot of the affinity purified recombinant dehydrogenase was incubated for 24 h at 22°C with 1 unit of enterokinase (Stratagene) in 100 µl (final volume) of 20 mM Tris-HCl buffer (pH 8.0) containing 50 mM NaCl and 2 mM CaCl₂.

**Extraction and assay of L-gulono-1,4-lactone dehydrogenase activity.** The Lgulono-1,4-lactone dehydrogenase activity was measured spectrophotometrically at 550 nm by following the L-gulono-1,4-lactone-dependent reduction of cytochrome c (Ôba et al., 1994).

**PAGE.** Proteins were separated by SDS/PAGE, using 12.5 % minigels and the buffer system described by Laemmli (1970). Gels were stained with Coomassie brilliant blue R-250.

**Protein determination.** Protein concentration was determined by the method of Bradford (1976), using BSA as standard.

### Example I: Heterologous expression and purification of the recombinant L-gulono-1,4-lactone dehydrogenase of Mycobacterium tuberculosis.

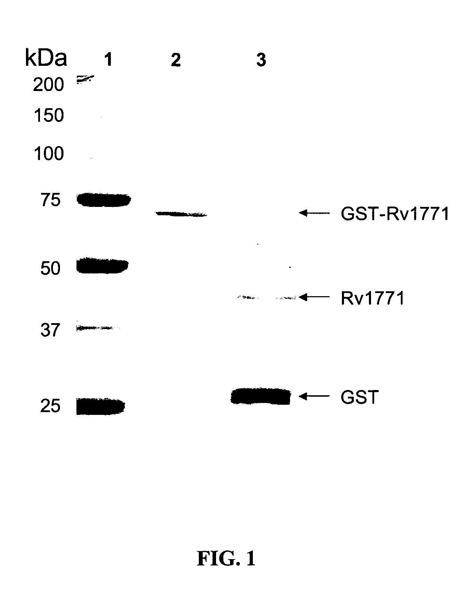

The L-gulono-1,4-lactone dehydrogenase DNA was cloned into pDEST15 vector with the GATEWAY system, and the obtained pDEST15-Gulox plasmid was used for expression of the recombinant glutathione S-transferase (GST) fusion protein in *E*. *coli.* The recombinant protein contained an engineered enterokinase cleavage site in the junction between the GST-tag and the mycobacterial dehydrogenase sequence. The recombinant dehydrogenase was obtained with a relatively low yield (0.2 mg per liter of culture). Even lower yields were obtained if Triton X-100 was omitted from the extraction buffer, thus suggesting that detergent helps to solubilize the protein and that the dehydrogenase might be membrane-associated. Analysis of the untreated affinity-purified enzyme by SDS-PAGE (Fig. 1) revealed the presence of one protein band at about 70 kDa. The identity of the protein was confirmed by enterokinase treatment. After digestion of the affinity-purified GST-tagged protein with enterokinase, the 70 kDa band disappeared and two new bands were seen on SDS-PAGE: the slower-migrating 45-kDa band and the 26-kDa band, that correspond to the mycobacterial Gulox dehydrogenase (Rv1771) and the glutathione Stransferase (GST), respectively. The observed molecular weights for the GST-tagged and the free Gulox dehydrogenase were slightly lower than those expected (74 and 48 kDa, respectively), presumably because of either a limited proteolysis or an abnormal migration.

### Example 2: Characterization of the recombinant L-gulono-1,4-lactone dehydrogenase of Mycobacterium tuberculosis.

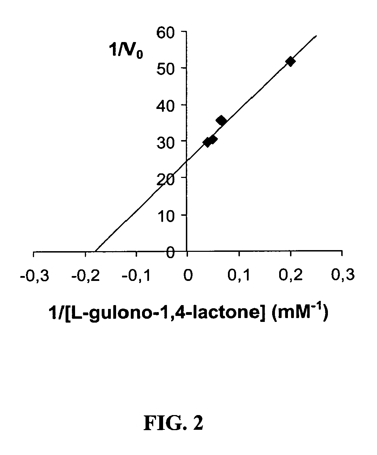

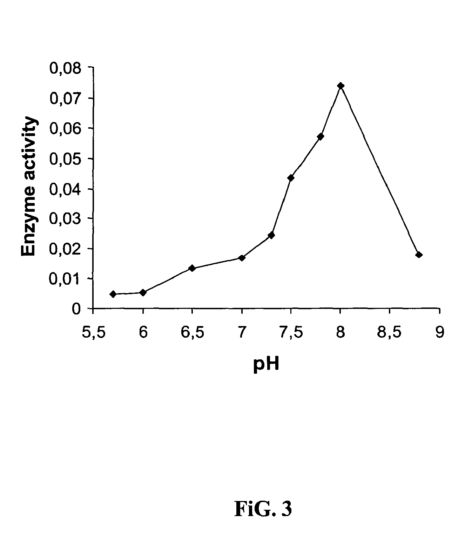

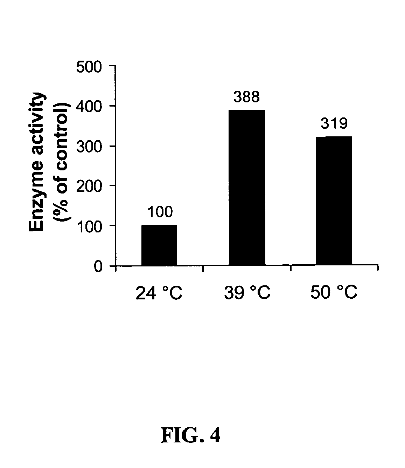

The L-gulono-1,4-lactone dehydrogenase of *M. tuberculosis* shows 32% of identity with the rat L-gulono-1,4-lactone oxidase (Table 1) and 22-24% of identity with the putative plant L-gulono-1,4-lactone dehydrogenases (Wolucka and Van Montagu, 2003). Similarly to the animal and plant L-gulono-1,4-lactone dehydrogenases/oxidases, the *M. tuberculosis* enzyme possesses a FAD-binding site (VGSGH₄₉S) at its N-terminus, containing a conserved histidine residue that is known to participate in the covalent binding of the FAD molecule in the rat enzyme (Nakagawa et al., 1975). Like the plant L-gulono-1,4-lactone and L-galactono-1,4-lactone dehydrogenases, the mycobacterial enzyme acts exclusively as a dehydrogenase because it can use cytochrome c as an electron acceptor but not molecular oxygen. In contrast to the animal and plant L-gulono-1,4-lactone dehydrogenases/oxidases, the mycobacterial enzyme is specific for L-gulono-1,4-lactone and has no activity towards L-galactono-1,4-lactone (Table 2). The recombinant dehydrogenase has a low apparent K_{*m*} value for L-gulono-1,4-lactone (5 mM) (Fig. 2 and Table 3) that is close to the value reported for the plant L-galactono-1,4-lactone dehydrogenase (3.3 mM) for the L-galactono-1,4-lactone substrate (Östergaard et al., 1997). The optimal pH is 8 (Fig. 3) and the temperature optimum is 39 °C (Fig. 4). Inhibition of the Gulox enzyme by N-ethylmaleimide, Cu²⁺ and Zn²⁺ (Table 4) points to the involvement of sylfhydryl group(s) in the catalytic activity, as observed also for the related L-galactono-1,4-lactone dehydrogenase (Ostergaard et al., 1997; Imai et al., 1998; Yabuta et al., 2000). The dehydrogenase requires trace amounts of a divalent metal ion for its activity because a complete inhibition of the enzyme was observed in the presence of 1 mM EDTA (Table 4). In agreement with the presence of a covalently-bound FAD molecule, the dehydrogenase activity was not affected by addition of exogenous FAD (Table 4).

In summary, the *M. tuberculosis* L-gulono-1,4-lactone dehydrogenase is a new and distinct member of the group of L-gulono-1,4-lactone dehydrogenase/oxidases that have been characterized until now.

### REFERENCES

Agius, F. et al. Engineering increased vitamin C levels in plants by overexpression of a D-galacturonic acid reductase. Nat Biotechnol 21, 177-81 (2003).
Allaudeen, H. S. & Ramakrishnan, T. Occurrence of ascorbic acid in mycobacterium tuberculosis H 37 R v. Indian J Exp Biol 9, 278-9 (1971).
Arrigoni, O., Paciolla, C. & De Gara, L. Inhibition of galactonolactone dehydrogenase activity by lycorine. Boll Soc Ital Biol Sper 72, 37-43 (1996).
Bardarov, S. et al. Specialized transduction: an efficient method for generating marked and unmarked targeted gene disruptions in Mycobacterium tuberculosis, M. bovis BCG and M. smegmatis. Microbiology 148, 3007-17 (2002).
Bleeg, H. S. & Christensen, F. Biosynthesis of ascorbate in yeast. Purification of L-galactono-1,4-lactone oxidase with properties different from mammalian L-gulonolactone oxidase. Eur J Biochem 127, 391-6 (1982).
Bradford, M. M. A rapid and sensitive method for the quantitation of microgram quantities of protein utilizing the principle of protein-dye binding. Anal Biochem 72, 248-54 (1976).
Castanon-Arreola, M. & Lopez-Vidal, Y. A second-generation anti TB vaccine is long overdue. Ann Clin Microbiol Antimicrob 3, 10 (2004).
Chen, Z., Young, T. E., Ling, J., Chang, S. C. & Gallie, D. R. Increasing vitamin C content of plants through enhanced ascorbate recycling. Proc Natl Acad Sci U S A 100, 3525-30 (2003).
Hondalus, M. K. et al. Attenuation of and protection induced by a leucine auxotroph of Mycobacterium tuberculosis. Infect Immun 68, 2888-98 (2000).
Hoshino, T., Matzinger, P.K., Ojima, S., Sugisawa, T. L-Gulono-gamma-lactone-dehydrogenase for producing vitamin C. U.S. Patent N° 5,250,428.
Huh, W. K. et al. Characterisation of D-arabinono-1,4-lactone oxidase from Candida albicans ATCC 10231. Eur JBiochem 225, 1073-9 (1994).
Huh, W. K. et al. D-Erythroascorbic acid is an important antioxidant molecule in Saccharomyces cerevisiae. Mol Microbiol 30, 895-903 (1998).
Huh, W. K., Kim, S. T., Kim, H., Jeong, G. & Kang, S. O. Deficiency of D-erythroascorbic acid attenuates hyphal growth and virulence of Candida albicans. Infect Immun 69, 3939-46 (2001).
Imai, T. et al. L-galactono-gamma-lactone dehydrogenase from sweet potato: purification and cDNA sequence analysis. Plant Cell Physiol 39, 1350-8 (1998).
Jackson, M. et al. Persistence and protective efficacy of a Mycobacterium tuberculosis auxotroph vaccine. Infect Immun 67, 2867-73 (1999).
Jain AK, Nessler CL. Metabolic engineering of an alternative pathway for ascorbic acid biosynthesis in plants. Mol. Breeding 6, 73-8 (2000).
Kiuchi, K., Nishikimi, M. & Yagi, K. Purification and characterization of L-gulonolactone oxidase from chicken kidney microsomes. Biochemistry 21, 5076-82 (1982).
Koshizaka, T., Nishikimi, M., Ozawa, T. & Yagi, K. Isolation and sequence analysis of a complementary DNA encoding rat liver L-gulono-gamma-lactone oxidase, a key enzyme for L-ascorbic acid biosynthesis. JBiol Chem 263, 1619-21 (1988).
Laemmli, U. K. Cleavage of structural proteins during the assembly of the head of bacteriophage T4. Nature 227, 680-5 (1970).
Loewus, F.A. Biosynthesis and metabolism of ascorbic acid in plants and of analogs of ascorbic acid in fungi. Phytochem. 52, 193-210 (1999).
Linster, C. L. & Van Schaftingen, E. Rapid stimulation of free glucuronate formation by non-glucuronidable xenobiotics in isolated rat hepatocytes. J Biol Chem 278, 36328-33 (2003).
McKenney, D. et al. Broadly protective vaccine for Staphylococcus aureus based on an in vivo-expressed antigen. Science 284, 1523-7 (1999).
Nakagawa, H., Asano, A. & Sato, R. Ascorbate-synthesizing system in rat liver microsomes. II. A peptide-bound flavin as the prosthetic group of L-gulono-gamma-lactone oxidase. J Biochem (Tokyo) 77, 221-32 (1975).
Nishikimi, M. L-Gulono-gamma-lactone oxidase (rat and goat liver). Methods Enzymol 62, 24-30 (1979).
Nishikimi, M. & Yagi, K. Biochemistry and molecular biology of ascorbic acid biosynthesis. Subcell Biochem 25, 17-39 (1996).
Nishikimi, M., Noguchi, E. & Yagi, K. Occurrence in yeast of L-galactonolactone oxidase which is similar to a key enzyme for ascorbic acid biosynthesis in animals, L-gulonolactone oxidase. Arch Biochem Biophys 191, 479-86 (1978).
Nishikimi, M., Fukuyama, R., Minoshima, S., Shimizu, N. & Yagi, K. Cloning and chromosomal mapping of the human nonfunctional gene for L-gulono-gamma-lactone oxidase, the enzyme for L-ascorbic acid biosynthesis missing in man. J Biol Chem 269, 13685-8 (1994).
Ôba, K., Fukui, M., Imai, Y., Iriyama, S. & Nogami, K. L-Galactono-g-lactone dehydrogenase: partial characterization, induction of activity and role in the synthesis of ascorbic acid in wounded potato tuber tissue. Plant Cell Physiol. 35, 473-8 (1994).
Ostergaard, J., Persiau, G., Davey, M. W., Bauw, G. & Van Montagu, M. Isolation of a cDNA coding for L-galactono-gamma-lactone dehydrogenase, an enzyme involved in the biosynthesis of ascorbic acid in plants. Purification, characterization, cDNA cloning, and expression in yeast. J Biol Chem 272, 30009-16 (1997).
Pavelka, M. S., Jr., Chen, B., Kelley, C. L., Collins, F. M. & Jacobs Jr, W. R., Jr. Vaccine efficacy of a lysine auxotroph of Mycobacterium tuberculosis. Infect Immun 71, 4190-2 (2003).
Pelicic, V. et al. Efficient allelic exchange and transposon mutagenesis in Mycobacterium tuberculosis. Proc Natl Acad Sci U S A 94, 10955-60 (1997).
Sambandamurthy, V. K. et al. A pantothenate auxotroph of Mycobacterium tuberculosis is highly attenuated and protects mice against tuberculosis. Nat Med 8, 1171-4 (2002).
Sambandamurthy, V. K. et al. Long-term protection against tuberculosis following vaccination with a severely attenuated double lysine and pantothenate auxotroph of Mycobacterium tuberculosis. Infect Immun 73, 1196-203 (2005).
Sampson, S. L. et al. Protection elicited by a double leucine and pantothenate auxotroph of Mycobacterium tuberculosis in guinea pigs. Infect Immun 72, 3031-7 (2004).
Shigeoka, S., Nakano, Y. & Kitaoka, S. Some properties and subcellular localization of L-gulono-γ-lactone dehydrogenase in Euglena gracilis z. Agric. Biol. Chem. 43, 2187-8 (1979).
Sugisawa, T., Ojima, S., Matzinger, P.K. & Hoshino, T. Isolation and characterization of a new vitamin C producing enzyme (L-gulono-gamma-lactone dehydrogenase) of bacterial origin. Biosci. Biotechnol. Biochem. 59, 190-6 (1995).
Valpuesta, V. & Botella, M. A. Biosynthesis of L-ascorbic acid in plants: new pathways for an old antioxidant. Trends Plant Sci 9, 573-7 (2004).
Wolucka, B. A. & Van Montagu, M. GDP-mannose 3',5'-epimerase forms GDP-L-gulose, a putative intermediate for the de novo biosynthesis of vitamin C in plants. J Biol Chem 278, 47483-90 (2003).
Yabuta, Y., Yoshimura, K., Takeda, T. & Shigeoka, S. Molecular characterization of tobacco mitochondrial L-galactono-gamma-lactone dehydrogenase and its expression in Escherichia coli. Plant Cell Physiol 41, 666-75 (2000).

**Table 1. The BLAST search (http://www.ncbi.nlm.nih.gov/entrez/query.fcgi) for homologues of the rat L-gulono-1,4-lactone oxidase (440 aa) in bacteria. Species belonging to Actinomycetales are underlined.**

| **Species** (accession number) | **Length (aa)** | **% of identity** |
|---|---|---|
| *Mycobacterium tuberculosis* (NP_216287) | 428 | 32 |
| *Mycobacterium bovis* (NP_855453) | 428 | 32 |
| *Streptomyces coelicolor* (NP_629980) | 445 | 31 |
| *Thermobifida fusca* (ZP_00059445) | 669 | 32 |
| *Streptomyces avermitilis* (NP_823585) | 439 | 31 |
| *Gibberella zeae* (XP_388870) | 514 | 30 |
| *Oceanobacillus iheyensis* (NP _692632) | 440 | 30 |
| *Bacillus cereus* (NP _830486) | 438 | 30 |
| *Bacillus anthracis* (NP_654628) | 437 | 29 |
| *Burkholderia cepacia (*ZP*_*00218082) | 455 | 30 |
| *Nostoc punctiforme* | 445 | 30 |
| *Pseudomonas aeruginosa* (NP_254014) | 442 | 27 |
| *Chromobacterium violaceum* (NP_900710) | 463 | 27 |
| *Caulobacter crescentus* (NP_420033) | 468 | 25 |
| *Sinorhizobium meliloti* (NP_384830) | 412 | 29 |
| *Mesorhizobium loti* (NP_108349) | 436 | 22 |

**Table 2. Substrate specificity of the recombinant GST-tagged L-gulono-1,4-lactone dehydrogenase of Mycobacterium tuberculosis.**

| **Substrate** | **Enzyme specific activity** |
|---|---|
| *(50 mM)* | *(mU*/*mg ofprotein)* |
| L-gulono-1,4-lactone | 66.7 |
| L-galactono-1,4-lactone | 0 |
| D-glucurono-3,6-lactone | 0 |
| D-glucuronic acid | 0 |
| D-arabinose | 0 |
| D-xylose | 0 |

**Table 3. Steady-state parameters of the recombinant L-gulono-1,4-lactone dehydrogenase of Mycobacterium tuberculosis.**

| **K**ₘ for L-gulono-1,4-lactone | **V**_{**max**} | **k**_{**cat**}**,** | **k**_{**cat**}**/K**_{**m**} |
|---|---|---|---|
| 5 mM | 2.44µmol·h⁻¹mg⁻¹ | 0.033 s⁻¹ | 0.0065 s⁻¹·mM⁻¹ |

**Table 4. Activity of the recombinant GST-tagged L-gulono-1,4-lactone dehydrogenase of Mycobacterium tuberculosis in the presence of effectors.**

| **Compound** | **Relative activity** |
|---|---|
| | (% of control) |
| No addition | 100 |
| N-ethylmaleimide (1 mM) | 0 |
| EDTA (1 mM) | 0 |
| CuCl₂ (1 mM) | 0 |
| ZnCl₂ (1 mM) | 0 |
| MnCl₂ (1 mM) | 79 |
| CaCl₂ (1 mM) | 91 |
| MgCl₂ (1 mM) | 98 |
| FAD (100 µM) | 94 |

### BRIEF DESCRIPTION OF THE FIGURES

Fig. 1. Purification of the recombinant L-gulono-1,4-lactone dehydrogenase (Rv1771) of *Mycobacterium tuberculosis.* SDS-PAGE of the affinity-purified recombinant GST-tagged dehydrogenase. Proteins were visualized by Coomassie blue staining. (1) Molecular mass standards. (2) Untreated affinity-purified GST-fusion protein. (3) Enterokinase (EK) cleavage products of the affinity-purified recombinant protein containing an engineered enterokinase cleavage site.
Fig. 2. Steady-state parameters of the recombinant GST-tagged L-gulono-1,4-lactone dehydrogenase of *Mycobacterium tuberculosis.* Double-reciprocal Lineweaver-Burke plot is shown. V₀ is µmol of L-gulono-1,4-lactone oxidized per min, per mg of the recombinant dehydrogenase. L-Gulono-1,4-lactone concentrations were from 5 to 25 mM. Cytochrome c concentration was 0.125 mM.
Fig. 3. pH dependence of the recombinant GST-tagged L-gulono-1,4-lactone dehydrogenase of *Mycobacterium tuberculosis.*
Fig. 4. Effect of temperature on the activity of the recombinant GST-tagged L-gulono-1,4-lactone dehydrogenase of *Mycobacterium tuberculosis.*

## Claims

1. The L-gulonolactone dehydrogenase, wherein said dehydrogenase comprises SEQ ID NO:2.

2. The L-gulonolactone dehydrogenase according to claim 1, wherein said dehydrogenase has the following characteristics: a. a high substrate specificity with L-gulono-1,4-lactone ; b. a lack of activity with L-galactono-1,4-lactone ; c. a Km value for L-gulono-1,4-lactone of about 5 mM or less ; d. an optimum pH of about 8 ; e. an optimum temperature of about 39°C ; f. inhibition by the metal ions Zn²⁺ and Cu²⁺ ; g. inhibition by N-ethylmaleimide.

3. An isolated nucleic acid molecule comprising a nucleic acid sequence selected from the group consisting of: a. a nucleic acid sequence that encodes an amino acid sequence that is at least about 70% identical to SEQ ID NO : 1, wherein said amino acid sequence has Lgulonolactone dehydrogenase activity; b. a nucleic acid sequence encoding a fragment of said amino acid sequence of (a), wherein said fragment has L-gulonolactone dehydrogenase activity; c. a nucleic acid sequence that is a probe or primer that hybridizes under high stringency conditions to a nucleic acid sequence of (a) or (b); and d. a nucleic acid sequence that is a complement of any of the nucleic acid sequences of (a)- (c).

4. A host cell transformed with a recombinant nucleic acid molecule comprising a nucleic acid sequence selected from the group consisting of: a. a nucleic acid sequence that encodes an amino acid sequence that is at least about 70% identical to SEQ ID NO: 2, wherein said amino acid sequence has L-gulonolactone dehydrogenase activity; and b. a nucleic acid sequence encoding a fragment of said amino acid sequence of (a), wherein said fragment has L-gulonolactone dehydrogenase activity.

5. The use of the L-gulono-1,4-lactone dehydrogenase according to any one of claims 1 to 4 to modulate ascorbic acid synthesis in a cell.

6. A method for the production of L-ascorbic acid by a host cell that is transformed with a recombinant nucleic acid molecule comprising a. a nucleic acid sequence encoding an amino acid sequence that is at least about 70% identical to SEQ ID NO : 2, wherein said amino acid sequence has L-gulonolactone dehydrogenase activity; and b. a nucleic acid sequence encoding a fragment of said amino acid sequence of (a), wherein said fragment has L-gulonolactone dehydrogenase activity.

7. The use of an L-gulonolactone dehydrogenase according to any one of claims 1 to 6 for in vitro synthesis of L-ascorbic acid.

8. A mycobacterium in the *Mycobacterium tuberculosis* complex, genetically engineered to comprise a deletion of a region controlling production of vitamin C or its precursor, wherein the *M. tuberculosis* exhibits attenuated virulence in a mammal when compared to the *M. tuberculosis* without the deletion.

9. The mycobacterium of claim 8, wherein at least one of the deleted regions comprises SEQ ID NO : 1.

10. A tuberculosis vaccine comprising the mycobacterium of any of claims 8 to 9 in a pharmaceutically acceptable excipient, wherein the vaccine is capable of protecting a mammal from a challenge by *M*. *tuberculosis.*

11. A tuberculosis vaccine comprising the mycobacterium of any of claims 8 to 9 in a pharmaceutically acceptable excipient, wherein the vaccine is capable of protecting a mammal from a challenge by *M. bovis.*

12. An antituberculosis drug comprising a compound which inhibits L-gulonolactone dehydrogenase according to any of claims 1 to 2.

13. A diagnostic method which uses L-gulonolactone dehydrogenase according to any one of claims 1 to 3.
